(19) Europäisches Patentamt
European Patent Office
Office européen des brevets

(11) Publication number: 0 113 992 B1

(12) # EUROPEAN PATENT SPECIFICATION

(45) Date of publication of patent specification: 22.04.87

(51) Int. Cl.$^4$: C 08 L 83/08, A 61 K 7/11, A 61 K 7/06

(21) Application number: 83307751.4

(22) Date of filing: 20.12.83

(54) **One-part moisture cured aminosiloxanes to set and condition hair.**

(30) Priority: 27.12.82 US 453637
27.12.82 US 453638

(43) Date of publication of application:
25.07.84 Bulletin 84/30

(45) Publication of the grant of the patent:
22.04.87 Bulletin 87/17

(84) Designated Contracting States:
DE FR GB

(56) References cited:
DE-A-2 034 727
US-A-3 832 203
US-A-4 344 763

(73) Proprietor: DOW CORNING CORPORATION
3901 S. Saginaw Road
Midland Michigan 48640 (US)

(72) Inventor: Homan, Gary Rex
2801 Daw Drive
Midland Michigan (US)
Inventor: Swihart, Terence John
1544 St. Marys Court
Essexville Michigan (US)

(74) Representative: Laredo, Jack Joseph et al
Elkington and Fife High Holborn House 52/54
High Holborn
London, WC1V 6SH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 113 992 B1

Courier Press, Leamington Spa, England.

**Description**

This invention relates to a composition, stable in the absence of moisture, consisting essentially of (A) a siloxane oligomer having at least one nitrogen-hydrogen bond; and (B) a readily hydrolyzable, anhydrous additive selected from the group consisting of titanates, zirconates, vanadates, germanates and mixtures thereof. The uniqueness of this invention is that the oligomer crosslinks upon exposure to moisture such as water vapor and reverts to a noncrosslinked oligomer after exposure to liquid water. Furthermore, the components can be stored together, in the absence of moisture, with no gelation.

Polymers crosslinked upon exposure to moisture and reverting to a polymer after exposure to water can find use in hair care applications and reversible rubber or elastomer applications. In hair care applications, the composition is applied to hair to set and condition. Upon washing, the composition reverts to a polymer and acts, again, as a hair conditioner.

A composition described in the article entitled "New Types of Hair Setting Sprays having Semi-Permanent Properties" authored by Fulvio Sardo in Volume 87, *American Cosmetics and Perfumery*, pages 43—46 (December, 1972), discloses the use of alkyltitanates in combination with silanol endblocked dimethylpolysiloxanes as a hair set. This composition has limited use as a conditioner, and there is no mention of the composition's capability of reversion to its polymer.

CA—A—1,112,181 discloses a hair set composition consisting essentially of aminoalkylalkoxysilanes and a titanate ester. This composition also has limited use as a hair conditioner with no mention of reversion capabilities.

US—A—3,832,203 discloses a composition for treating leather comprising an organic solvent solution of (A) a titanate or zirconate, a copolymer of trimethylsiloxane and $SiO_2$ units, a polysiloxane, and (B) an aminoalkyl substituted silane or siloxane. There is no mention of this composition being reversible and parts (A) and (B) cannot be stored together.

US—A—4,388,437 discloses an organopolysiloxane composition, used for treating fibers, consisting essentially of an organopolysiloxane containing amine functionality, a surfactant, a titanate, zirconate or germanate, an organic acid, and water. The surfactant is needed to emulsify the siloxane. The acid is essential to adjust the pH to a desired range to control the rate of adsorption of the emulsion on the fiber. There is no mention of this composition's reversion capabilities.

An object of this invention is to provide a composition stable in the absence of moisture, crosslinkable upon exposure to moisture, and revertable to a polymer after exposure to water. A practical application for this phenomenon provides a process for concurrently setting and conditioning hair comprising (I) imposing a desired configuration on the hair; (II) coating the hair with a composition comprising (A) an organosiloxane oligomer having an organic substituent containing a nitrogen-hydrogen bond; (B) a readily hydrolyzable, anhydrous compound selected from the group consisting of organic titanates, organic zirconates, organic vanadates, organic germanates, and mixtures thereof; and (C) an anhydrous organic solvent for (A) and (B); and (III) exposing the coated hair to air.

The present invention provides a composition, stable in the absence of moisture, consisting essentially of (A) a siloxane oligomer having at least one nitrogen-hydrogen bond wherein the units containing the nitrogen-hydrogen bond are selected from the group consisting of

$$O\overset{\overset{R}{|}}{Si}C_mH_{2m}NR'_2, \qquad O_{0.5}\overset{\overset{R_2}{|}}{Si}C_mH_{2m}NR'_2,$$

$$O\overset{\overset{R}{|}}{Si}C_mH_{2m}NR'C_nH_{2n}NR'_2, \qquad O_{0.5}\overset{\overset{R_2}{|}}{Si}C_mH_{2m}NR'C_nH_{2n}NR'_2,$$

$$O_{1.5}SiC_mH_{2m}NR'_2, \qquad O_{1.5}SiC_mH_{2m}NR'C_nH_{2n}NR'_2,$$

$$O\overset{\overset{R}{|}}{Si}C_mH_{2m}\overset{\overset{O}{\|}}{C}NR'_2, \qquad O_{0.5}\overset{\overset{R_2}{|}}{Si}C_mH_{2m}\overset{\overset{O}{\|}}{C}NR'_2,$$

$$O\overset{\overset{R}{|}}{Si}C_mH_{2m}\overset{\overset{O}{\|}}{C}NR'C_nH_{2n}\overset{\overset{O}{\|}}{C}NR'_2, \qquad O_{0.5}\overset{\overset{R_2}{|}}{Si}C_mH_{2m}\overset{\overset{O}{\|}}{C}NR'C_nH_{2n}\overset{\overset{O}{\|}}{C}NR'_2,$$

$$O_{1.5}SiC_mH_{2m}\overset{\overset{O}{\|}}{C}NR'_2, \qquad O_{1.5}SiC_mH_{2m}\overset{\overset{O}{\|}}{C}NR'C_nH_{2n}\overset{\overset{O}{\|}}{C}NR'_2,$$

and mixtures thereof wherein R is selected from the group consisting of monovalent hydrocarbon radicals, substituted monovalent hydrocarbon radicals and hydrogen, R' is selected from the group consisting of

hydrogen, monovalent hydrocarbon radicals, and substituted monovalent hydrocarbon radicals; wherein at least one R′ per siloxy unit is hydrogen; and *m* and *n* have values of 1 to 4, and (B) a readily hydrolyzable, anhydrous additive selected from the group consisting of titanates, zirconates, vanadates, germanates and mixtures thereof.

It should be understood that the divalent radicals represented as $C_mH_{2m}$ and $C_nH_{2n}$ can be either linear or branched hydrocarbon radicals. Specific examples of suitable divalent hydrocarbon radicals represented by $C_mH_{2m}$ and $C_nH_{2n}$ in the above formulae include

$$-(CH_2)_2-,\ -(CH_2)_3-,\ -(CH_2)-,\ -CH_2CHCH_3CH_2-$$

and

$$-CH_2CH_2\underset{}{\overset{CH_3}{\overset{|}{C}}}H-.$$

Specific examples of suitable monovalent hydrocarbon and substituted monovalent hydrocarbon radicals for R, and R′, are alkyl radicals such as the methyl, ethyl, propyl, butyl, amyl, cyclohexyl, decyl, dodecyl, and octadecyl radicals; alkenyl radicals such as the vinyl and allyl radicals; aryl radicals such as the phenyl and biphenyl radicals; alkaryl and aralkyl radicals such as the tolyl and benzyl radicals; and the corresponding substituted hydrocarbon radicals such as the chloropropyl, 3,3,3-trifluoropropyl, dichlorophenyl, cyanobutyl, and nitrophenyl radicals.

A siloxane polymer having the nitrogen-hydrogen bond units in the siloxy unit having the general formula

$$O\overset{R}{\overset{|}{Si}}C_mH_{2m}NR'C_nH_{2n}NR'_2$$

wherein R is a monovalent hydrocarbon radical, R′ is hydrogen, *m* has a value of 3 or 4 and *n* has a value of 2 is preferred. R being an alkyl radical is further preferred with methyl being optimal.

The other units of the siloxane oligomer are selected from the group consisting of $RSiO_{3/2}$, $R_2SiO$, and $R_3SiO_{1/2}$ units wherein R is as defined above. A siloxane oligomer composed primarily of $R_2SiO$ units is preferred. Furthermore the endblocking and the structure of the oligomer is not critical for the purpose of this invention as long as the siloxane oligomer contains at least one nitrogen-hydrogen bond. Also, the siloxane oligomer containing at least one nitrogen-hydrogen bond can be blended with other functional siloxanes or non-functional siloxanes, such as dimethylsiloxane or dimethylcyclosiloxane. However, a greater amount of amine or amide present leads to greater crosslinking.

A siloxane oligomer having the general formula

$$R''_3Si(O\underset{R_2}{\underset{|}{Si}})_a(O\overset{R}{\overset{|}{\underset{C_mH_{2m}NHC_nH_{2n}NH_2}{\underset{|}{Si}}}})_bOSiR''_3$$

wherein R is as defined above, R″ is selected from the group consisting of monovalent hydrocarbon radicals, and substituted monovalent hydrocarbon radicals, and *a*+*b* has a value of 1 to 2000 with *a* having a value of 0 to 1999, and *b* having a value of 1 to 2000, is preferred. Specific examples of suitable monovalent hydrocarbon radicals and substituted monovalent hydrocarbon radicals for R″ are as defined above for R, and R′. But, further preference is given to R″ being a monovalent hydrocarbon radical, with R″ being an alkyl radical being further preferred, and methyl being optimal. The value of *a*+*b* having a value of 50 to 400 with *a* having a value of 49 to 399 and *b* having a value of 1 to 30 is also further preferred. A siloxane oligomer selected from the group consisting of

$$(CH_3)_3SiO[(CH_3)_2SiO]_{98}(CH_3\underset{(CH_2)_3NH(CH_2)_2NH_2}{\underset{|}{Si}}O)_2Si(CH_3)_3$$

$$(CH_3)_3SiO[(CH_3)_2SiO]_{98}(CH_3\underset{CH_2CHCH_3CH_2NHCH_2CH_2NH_2}{\underset{|}{Si}}O)_2Si(CH_3)_3,\ \text{and}$$

$$(CH_3)_3SiO[(CH_3)_2SiO]_{95}(CH_3\underset{(CH_2)_3NH(CH_2)_2NH_2}{\underset{|}{Si}}O)_5Si(CH_3)_3$$

is further preferred.

So far as is known at this time, any readily hydrolyzable, anhydrous additive selected from the group consisting of titanates, zirconates, vanadates, germanates and mixtures thereof can be employed for the purpose of this invention. Specific examples of suitable organic zirconates include octyleneglycolzirconate, tetra-2-ethylhexylzirconate, tetraisopropylzirconate, tertiarybutyltrimethylzirconate and mixtures thereof. Specific examples of suitable organic titanates include tetraethyltitanate, tetradecyltitanate, tetra-2-ethylhexyltitanate, tetradodecyltitanate, tetraisopropyltitanate, tetrabutyltitanate and mixtures thereof. Specific examples of suitable organic germanates include tetraethylgermanate, tetraisopropylgermanate, and tetradecylgermanate. Specific examples of suitable organic vanadates include vanadium triisopropoxide oxide and vanadium tri-n-propoxide oxide.

Titanates are preferred, however, Organic titanates selected from the group consisting of alkyl titanates and titanium chelates are further preferred. Alkyl titanates have the general formula $Ti(OD)_4$ wherein D is a monovalent hydrocarbon radical or a substituted monovalent hydrocarbon radical.

Titanium chelates have the general formulae

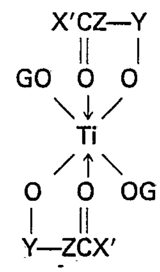

and

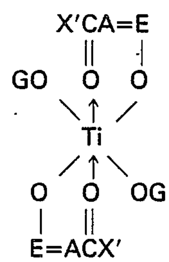

wherein G is a monovalent hydrocarbon or substituted monovalent hydrocarbon radical, X′ is selected from the group consisting of hydrogen, $-OCH_2CH_3$, —OH, monovalent hydrocarbon radicals, and substituted monovalent hydrocarbon radicals, Z and Y are divalent hydrocarbon or substituted divalent hydrocarbon radicals, and A and E are trivalent or substituted trivalent hydrocarbon radicals.

Specific examples of suitable monovalent hydrocarbon or substituted monovalent hydrocarbon radicals for D, X′, and G are alkyl radicals such as the methyl, ethyl, propyl, butyl, amyl, cyclohexyl, decyl, dodecyl, and octadecyl radicals; alkenyl radicals such as the vinyl and allyl radicals; aryl radicals such as the phenyl and biphenyl radicals; alkaryl and aralkyl radicals such as the tolyl and benzyl radicals; and the corresponding substituted hydrocarbon radicals such as the chloropropyl, 3,3,3-trifluoropropyl, dichlorophenyl, cyanobutyl, and nitrophenyl radicals.

The hydrocarbon and substituted hydrocarbon radicals for Y and Z are divalent radicals. Though the above examples of suitable hydrocarbon and substituted hydrocarbon radicals for D, X′ and G are in monovalent form, similar divalent radicals are suitable for Y and Z. Specific examples of suitable divalent hydrocarbon radicals include

$$-(CH_2)_3-,\ -(CH_2)_4-,\ -(CH_2)_6-,\ \text{and}\ -CH_2CHCH_3CH_2-.$$

The hydrocarbon and substituted hydrocarbon radicals for A and E are trivalent radicals. Though the above examples of suitable hydrocarbon and substituted hydrocarbon radicals for D, X′, and G are in monovalent form, similar trivalent radicals are suitable for A and E. Specific examples of suitable trivalent hydrocarbon radicals include

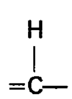 and 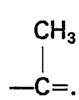.

Specific examples of suitable titanates having the general formula $Ti(OD)_4$ include tetraisopropyltitanate, tetra-n-butyltitanate and tetrakis(2-ethylhexyl)titanate. Specific examples of suitable titanates having the general formula

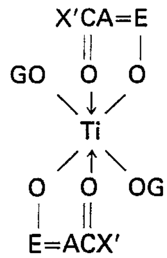

include titanium acetylacetonate and titanium ethyl acetylacetonate.

Titanates having the general formula $Ti(OD)_4$ wherein D is an alkyl radical are preferred. Further preference is given to the titanate having the general formula $Ti(OD)_4$ wherein D is selected from the group consisting of isopropyl, 2-ethylhexyl, and n-butyl radicals.

If desired the composition may also contain an anhydrous organic solvent. Examples of suitable solvents include aliphatic hydrocarbon and aliphatic halogenated hydrocarbon solvents such as hexane, heptane, and 1,1,1-trichloroethane; and aromatic hydrocarbon solvents such as toluene and xylene. The solvent, 1,1,1-trichloroethane, is preferred.

Compositions containing 0.97 to 90 percent by weight of the siloxane, 0.03 to 15 percent by weight of the metallic additive, and 1 to 99 percent by weight of a solvent are preferred. Compositions containing from 3 to 20 percent by weight of the siloxane, 0.1 to 3 percent by weight of the metallic additive and 77 to 96.9 percent by weight of a solvent are further preferred.

If desired, the composition may also contain 0.01 to 50 percent by weight of an alkoxy silane having at least two alkoxy groups per silicon for added durability. Specific examples of suitable alkoxy silanes include methyl trimethoxysilane and a silane having the general formula

$$NH_2CH_2CH_2NHCH_2CH_2CH_2Si(OCH_3)_3.$$

In addition to the essential ingredients, the composition of this invention may include minor quantities of optional materials which are added for specific purposes. Such other ingredients include, but are not limited to, medicaments such as anti-dandruff compounds, solvents, perfumes, sequestering agents, conditioning agents such as quaternary ammonium compounds, thickening agents, fillers, opacifiers, anti-static agents, and antimicrobial preservatives, of which silicone is one.

The methods of preparation of the components of the present invention are well known to those skilled in the art and detailed elsewhere in the literature. Hence, no time or space need be devoted here to a repetition of such information. As far as is known at this time, the order or method of mixing the components employed is not critical for the purpose of this invention.

Suitable uses for this composition are in the field of hair care and anywhere there is need for a reversible rubber or elastomer. In the field of hair care, preference is given to applying the composition to preset and dried hair. The composition can be applied to the hair in any suitable form such as in thickened form by hand, in viscous form, by aerosol, or by pump spray. The composition after curing on the hair acts to hold any set in the hair even when exposed to atmospheric humidity, but when the hair is contacted directly with water such as by washing, the set is removed from the hair. Washing hair with a shampoo rather than just water is even more effective in removing the set from the hair. However, sufficient composition remains on the hair to act as a conditioner for the wet hair. Furthermore, if the hair is reset and dried, the composition again acts to hold the set in the hair although to a somewhat lesser extent than after the initial application of the composition. Although not intending to be bound by this theory, applicant believes that the cured composition when contacted directly with water such as by washing, reverts to the noncrosslinked, uncured polysiloxane which acts as the conditioner for the wet hair. When the hair is again dried however, applicant believes that the composition that remains on the hair again cures to sufficient extent to improve the set holding properties of the hair. The term "hair" as used in the present invention includes treated and untreated human hair, animal hair, and any type of fiber that needs gloss, ease of combing, set hold, feel of fullness, protection from the elements such as sun, and reduced fly-away. Treated hair includes hair that is chemically changed and/or damaged by permanents and dyes. Setting hair means to curl, wave, control, or straighten air.

Now in order that those skilled in the art may better understand how the present invention can be practiced, the following examples are given by way of illustration and not by way of limitation. All parts and percents referred to herein are by weight unless otherwise specified.

Example 1

The following formulations were prepared by weighing the components into a 4 oz. (113 g) bottle, capping, and shaking:

Formulation A

10 grams $(CH_3)_3SiO[(CH_3)_2SiO]_{98}(CH_3SiO)_2Si(CH_3)_3$

|

$(CH_2)_3NH(CH_2)_2NH_2$

0.5 grams tetraisopropyltitanate

Formulation B

100 grams $(CH_3)_3SiO[(CH_3)_2SiO]_{98}(CH_3SiO)_2Si(CH_3)_3$

|

$(CH_2)_3NH(CH_2)_2NH_2$

7.5 grams tetraisopropyltitanate

Formulation C

100 grams $(CH_3)_3SiO[(CH_3)_2SiO]_{98}(CH_3SiO)_2Si(CH_3)_3$

|

$(CH_2)_3NH(CH_2)_2NH_2$

7.5 grams tetrakis(2-ethylhexyl)titanate

Formulation D

100 grams $(CH_3)_3SiO[(CH_3)_2SiO]_{95}(CH_3SiO)_5Si(CH_3)_3$

|

$(CH_2)_3NH(CH_2)_2NH_2$

7.5 grams tetraisopropyltitanate

Formulation E

90 percent by weight

$(CH_3)_3SiO[(CH_3)_2SiO]_{98}(CH_3SiO)_2Si(CH_3)_3$

|

$CH_2CHCH_3CH_2NHCH_2CH_2NH_2$

10 percent by weight tetraisopropyltitanate

Formulation F

90 percent by weight

$(CH_3)_3SiO[(CH_3)_2SiO]_{98}(CH_3SiO)_2Si(CH_3)_3$

|

$CH_2CHCH_3CH_2NHCH_2CH_2NH_2$

10 percent by weight tetra-n-butyltitanate

Formulation G

90 percent by weight

$(CH_3)_3SiO[(CH_3)_2SiO]_{98}(CH_3SiO)_2Si(CH_3)_3$

|

$CH_2CHCH_3CH_2NHCH_2CH_2NH_2$

10 percent by weight tetrakis(2-ethylhexyl)titanate

Formulation H

90 percent by weight

$(CH_3)_3SiO[(CH_3)_2SiO]_{98}(CH_3SiO)_2Si(CH_3)_3$

|

$CH_2CHCH_3CH_2NHCH_2CH_2NH_2$

10 percent by weight titanium acetylacetonate

Formulation I

90 percent by weight

$(CH_3)_3SiO[(CH_3)_2SiO]_{98}(CH_3SiO)_2Si(CH_3)_3$

|

$CH_2CHCH_3CH_2NHCH_2CH_2NH_2$

10 percent by weight titanium ethyl acetylacetonate

Formulation J

10 grams $(CH_3)_3SiO[(CH_3)_2SiO]_{98}(CH_3SiO)_2(CH_3)_3$

|

$(CH_2)_3N(CH_3)_2$

0.5 grams tetraisopropyltitanate

Formulation K

10 grams $(CH_3)_3SiO[(CH_3)_2SiO]_{98}(CH_3SiO)_2Si(CH_3)_3$

|

$(CH_2)_3NH(CH_2)_2NH_2$

0.5 grams tetraoctylene glycol titanium chelate

Formulation L

10 grams $(CH_3)_3SiO[(CH_3)_2SiO]_{98}(CH_3SiO)_2Si(CH_3)_3$

|

$(CH_2)_3NH(CH_2)_2NH_2$

0.75 grams triethanolamine titanium chelate

Formulation M

90 percent by weight

$(CH_3)_3SiO[(CH_3)_2SiO]_{98}(CH_3SiO)_2Si(CH_3)_3$

|

$CH_2CHCH_3CH_2NHCH_2CH_2NH_2$

10 percent by weight tetraoctylene glycol titanium chelate

Formulation N

90 percent by weight

$(CH_3)_3SiO[(CH_3)_2SiO]_{98}(CH_3SiO)_2Si(CH_3)_3$

|

$CH_2CHCH_3CH_2NHCH_2CH_2NH_2$

10 percent by weight triethanolamine titanium chelate

After mixing, the fluids were clear and colorless and there were no apparent increases in viscosity. The caps were removed and a portion of the fluid poured into aluminum cups exposed to atmospheric air. The results are as follows:

| Formulation | Surface skin time, 23°C | Tack free time, 23°C |
|---|---|---|
| A | 3 secs | 12 secs |
| B | immediate | immediate |
| C | 10 secs | 2 min |
| D | 2 secs | 10 secs |
| E | 1 sec | 1 sec |
| F | 1 secs | 20 secs |
| G | 5 min | 21 min |
| H | 12 secs | 4.5 min |
| I | 45 secs | 4 min |
| J* | none | — |
| K* | none | — |
| L* | none | — |
| M* | none | — |
| N* | none | — |

*Included for purposes of comparison

After 24 hours of exposure, formulations A, B, C, D, E, F, G, H, and I were cured to a thickness of about 1/16 inch (1,6 mm) while the materials kept in the capped bottles were still unchanged.

Formulation J indicates the importance of the N—H bond. The composition was exposed to air for 24 hours and showed no surface skin.

Formulations, K, L, M, and N indicate the importance of the titanate being readily hydrolyzable. The tetraoctylene glycol titanium chelate and the triethanolamine titanium chelate used in the formulations are not readily hydrolyzable. These formulations were also exposed to air for 24 hours and showed no evidence of forming a surface skin. The absence of surface skin in these formulations after 24 hours of exposure to the atmosphere is a simple test that can be employed to distinguish organic titanates, zirconates, germanates, and vanadates that are not readily hydrolyzable.

Formulation D was shelf-aged for 16 days and then the above process was repeated giving similar results. A portion of its cured rubber, 1/16 inch (1.6 mm) thick, was also heat aged 3 days at 100°C with no apparent adverse effects. Another portion of this cured rubber was immersed in tap water and became soft after 3 hours. After 3 days of immersion, the cured rubber reverted to the polymer and a white precipitate.

Example 2

A formulation was evaluated for the ability of the present invention to hold a set to hair. The formulation consisted of 10 percent by weight of

$$(CH_3)_3SiO[(CH_3)_2SiO]_{98}(CH_3\underset{\displaystyle (CH_2)_3NH(CH_2)_2NH_2}{\underset{|}{Si}}O)_2Si(CH_3)_3$$

1 percent by weight of tetraisopropyltitanate, and 89 percent by weight of 1,1,1-trichloroethane. The components were mixed together and the formulation was then placed in an aerosol can with 50 $cm^3$ of dichlorodifluoromethane propellant.

The test consisted of spraying the formulation onto undyed, preset, and dried hair and then exposing the hair to a water mist. An untreated specimen, also undyed, preset, and dried, was also exposed to the water mist.

The silicone treated hair held its set while the control completely lost its set after 24 hours exposure to atmospheric air.

Example 3

The following formulations were prepared by weighing the components into a 1 ounce (28.35 g) bottle, capping and shaking. The siloxane employed in the formulations had the general formula of

$$(CH_3)_3SiO[(CH_3)_2SiO]_{98}(CH_3\underset{\displaystyle (CH_2)_3NH(CH_2)_2NH_2}{\underset{|}{Si}}O)_2Si(CH_3)_3$$

Nine g of the siloxane and 1 g of the additive were employed. Vanadium triisopropoxide oxide, zirconium-n-propoxide, and germanium butoxide were the additives employed in Formulations A, B, and C, respectively.

After mixing, the formulations were clear with no apparent increase in viscosity. The caps were then removed and a portion poured into aluminum cups exposed to atmospheric moisture. The results are as follows:

| Formulation | Surface skin time, 23°C | Tack free time, 23°C |
|---|---|---|
| A | 2 s | 5 s |
| B | 15 s | 85 s |
| C | 3 s | 20 s |

After 24 hours of exposure, formulations A, B, and C were cured to a thickness of about 1/16 inch (1.6 mm) while the material kept in the capped bottles was still in an unchanged, fluid state.

**Claims**

1. A composition, stable in the absence of moisture, consisting essentially of (A) a siloxane oligomer having at least one nitrogen-hydrogen bond wherein the units containing the nitrogen-hydrogen bond are selected from the group consisting of

$$\begin{array}{l} \;\;R \\ \;\;| \\ OSiC_mH_{2m}NR'_2, \end{array} \qquad \begin{array}{l} \;\;\;\;R_2 \\ \;\;\;\;| \\ O_{0.5}SiC_mH_{2m}NR'_2, \end{array}$$

$$\begin{array}{l} \;\;R \\ \;\;| \\ OSiC_mH_{2m}NR'C_nH_{2n}NR'_2, \end{array} \qquad \begin{array}{l} \;\;\;\;R_2 \\ \;\;\;\;| \\ O_{0.5}SiC_mH_{2m}NR'C_nH_{2n}NR'_2, \end{array}$$

$$O_{1.5}SiC_mH_{2m}NR'_2, \qquad O_{1.5}SiC_mH_{2m}NR'C_nH_{2n}NR'_2,$$

$$\begin{array}{l} \;\;R \qquad\quad O \\ \;\;| \qquad\quad \| \\ OSiC_mH_{2m}CNR'_2, \end{array} \qquad \begin{array}{l} \;\;\;\;R_2 \qquad O \\ \;\;\;\;| \qquad\;\; \| \\ O_{0.5}SiC_mH_{2m}CNR'_2, \end{array}$$

$$\begin{array}{l} \;\;R \qquad\quad O \qquad\qquad\quad O \\ \;\;| \qquad\quad \| \qquad\qquad\quad \| \\ OSiC_mH_{2m}CNR'C_nH_{2n}CNR'_2, \end{array} \qquad \begin{array}{l} \;\;\;\;R_2 \qquad O \qquad\qquad\quad O \\ \;\;\;\;| \qquad\;\; \| \qquad\qquad\quad \| \\ O_{0.5}SiC_mH_{2m}CNR'C_nH_{2n}CNR'_2, \end{array}$$

$$\begin{array}{l} \qquad\qquad\quad O \\ \qquad\qquad\quad \| \\ O_{1.5}SiC_mH_{2m}CNR'_2, \end{array} \qquad \begin{array}{l} \qquad\qquad\quad O \qquad\qquad\quad O \\ \qquad\qquad\quad \| \qquad\qquad\quad \| \\ O_{1.5}SiC_mH_{2m}CNR'C_nH_{2n}CNR'_2, \end{array}$$

and mixtures thereof wherein R is selected from the group consisting of monovalent hydrocarbon radicals, substituted monovalent hydrocarbon radicals and hydrogen, R' is selected from the group consisting of hydrogen, monovalent hydrocarbon radicals, and substituted monovalent hydrocarbon radicals; wherein at least one R' per siloxy unit is hydrogen; and *m* and *n* have values of 1 to 4, the other units of the siloxane oligomer being selected from the group consisting of $RSiO_{3/2}$, $R_2SiO$, and $R_3SiO_{1/2}$ units wherein R is as defined above, and (B) a readily hydrolyzable, anhydrous additive selected from the group consisting of titanates, zirconates, vanadates, germanates and mixtures thereof.

2. A composition as defined in Claim 1, wherein the additive (B) is a titanate.

3. A composition as defined in Claim 2, wherein the units containing the nitrogen-hydrogen bond in the siloxane oligomer (A) are

$$\begin{array}{l} \;\;R \\ \;\;| \\ OSiC_mH_{2m}NR'C_nH_{2n}NR'_2, \end{array}$$

wherein R is a monovalent hydrocarbon radical, R' is hydrogen, *m* has a value of 3 or 4, and *n* has a value of 2; and the titanate (B) is an alkyl titanate having the general formula $Ti(OD)_4$ wherein D is an alkyl radical.

4. A composition as defined in Claim 3, wherein the siloxane oligomer (A) has the general formula:

$$\begin{array}{l} \qquad\qquad\qquad\quad R \\ \qquad\qquad\qquad\quad | \\ R''_3Si(OSi)_a(OSi)_bOSiR''_3 \\ \qquad\quad\;\; | \qquad\;\; | \\ \qquad\quad\; R_2 \quad C_mH_{2m}NHC_nH_{2n}NH_2 \end{array}$$

wherein R is an alkyl radical; R'' is selected from the group consisting of monovalent hydrocarbon radicals and substituted monovalent hydrocarbon radicals, and $a+b$ has a value of 1 to 2000 with *a* having a value of 0 to 1999 and *b* having a value of 1 to 2000.

5. A composition as defined in Claim 4, wherein the siloxane oligomer (A) is selected from the group consisting of:

$$\begin{array}{l} (CH_3)_3SiO[(CH_3)_2SiO]_{98}(CH_3SiO)_2Si(CH_3)_3 \\ \qquad\qquad\qquad\qquad\qquad\quad | \\ \qquad\qquad\qquad\qquad\qquad\quad (CH_2)_3NH(CH_2)_2NH_2 \end{array}$$

$$\begin{array}{l} (CH_3)_3SiO[(CH_3)_2SiO]_{98}(CH_3SiO)_2Si(CH_3)_3 \\ \qquad\qquad\qquad\qquad\qquad\quad | \\ \qquad\qquad\qquad\qquad\qquad\quad CH_2CHCH_3CH_2NHCH_2CH_2NH_2, \text{ and} \end{array}$$

$$\begin{array}{l} (CH_3)_3SiO[(CH_3)_2SiO]_{95}(CH_3SiO)_5Si(CH_3)_3 \\ \qquad\qquad\qquad\qquad\qquad\quad | \\ \qquad\qquad\qquad\qquad\qquad\quad (CH_2)_3NH(CH_2)_2NH_2 \text{ and,} \end{array}$$

the alkyl radical D in component (B) is selected from the group consisting of isopropyl, 2-ethylhexyl, and n-butyl radicals.

6. A process for concurrently setting and conditioning hair comprising:

(I) imposing a desired configuration on the hair;
(II) coating the hair with a composition according to any one of claims 1 to 5; and
(III) exposing the coated hair to air.

**Patentansprüche**

1. In Abwesenheit von Feuchtigkeit stabile Zusammensetzung, im wesentlichen bestehend aus (A) einem Siloxanoligomer mit mindestens einer Stickstoff-Wasserstoffbindung, wobei die Einheiten, die die Stickstoff-Wasserstoffbindung enthalten aus der aus

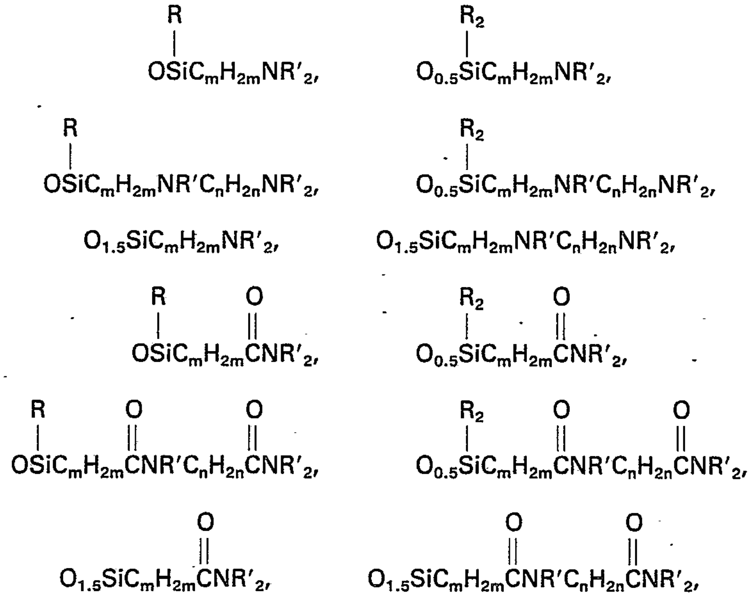

bestehenden Gruppe und Mischungen derselben ausgewählt sind, in denen R aus der Gruppe bestehend aus einwertigen Kohlenwasserstoffresten, substituierten einwertigen Kohlenwasserstoffresten und Wasserstoff ausgewählt ist und R′ aus der Gruppe bestehend aus Wasserstoff, einwertigen Kohlenwasserstoffresten, substituierten einwertigen Kohlenwasserstoffresten ausgewählt ist, wobei mindestens ein R′ pro Siloxygruppe Wasserstoff ist und m und n Werte zwischen 1 und 4 aufweisen, die anderen Einheiten der Siloxanoligomere sind aus der Gruppe bestehend aus $RSiO_{3/2}$, $R_2SiO$ und $R_3SiO_{1/2}$-Einheiten, in der R die gleiche Bedeutung wie zuvor hat, ausgewählt und

(B) einem leicht hydrolisierbaren wasserfreien Zusatz aus der Gruppe bestehend aus Titanaten, Zirkonaten, Vanadaten, Germanaten und Mischungen derselben.

2. Zusammensetzung nach Anspruch 1, dadurch gekennzeichnet, daß der Zusatz (B) ein Titanat ist.

3. Zusammensetzung nach Anspruch 2, dadurch gekennzeichnet, daß die Stickstoff-Wasserstoffbindung enthaltenden Einheiten im Siloxanoligomer (A)

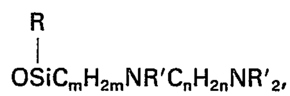

sind, in denen R ein einwertiger Kohlenwasserstoffrest und R′ Wasserstoff ist, m einen Wert von 3 oder 4 aufweist und n den Wert von 2 hat,
daß Titanat (B) ein Alkyltitanat der allgemeinen Formel $Ti(OD)_4$ ist, in der D eine Alkylgruppe ist.

4. Zusammensetzung nach Anspruch 3, dadurch gekennzeichnet, daß das Siloxanoligomer (A) die allgemeine Formel

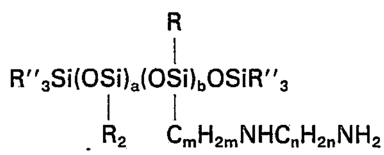

aufweist, in der R eine Alkylgruppe ist, R″ ausgewählt ist aus der Gruppe im wesentlichen bestehend aus einwertigen Kohlenwasserstoffresten und substituierten einwertigen Kohlenwasserstoffresten und a+b einen Wert von 1 bis 2000 hat mit a einen Wert von 0 bis 1999 und b einen Wert von 1 bis 2000.

5. Zusammensetzung nach Anspruch 4, dadurch gekennzeichnet, daß das Siloxanoligomer (A) ausgewählt ist aus der Gruppe im wesentlichen bestehend aus

$$(CH_3)_3SiO[(CH_3)_2SiO]_{98}(CH_3SiO)_2Si(CH_3)_3$$
|
$$(CH_2)_3NH(CH_2)_2NH_2$$

$$(CH_3)_3SiO[(CH_3)_2SiO]_{98}(CH_3SiO)_2Si(CH_3)_3$$
|
$$CH_2CHCH_3CH_2NHCH_2CH_2NH_2,$$ and

$$(CH_3)_3SiO[(CH_3)_2SiO]_{95}(CH_3SiO)_5Si(CH_3)_3$$
|
$$(CH_2)_3NH(CH_2)_2NH_2$$

und die Alkylgruppe D in dem Bestandteil (B) ausgewählt ist aus der Gruppe Isopropyl, 2-Ethylhexyl und n-Butylgruppen.

6. Verfahren zum gleichzeitigen Legen und Konditionieren von Haar durch

(I) Einrichten der gewünschten Form des Haares,
(II) Beschichten des Haares mit einer Zusammensetzung nach den Ansprüchen 1 bis 5 und
(III) Belüften des beschichteten Haares.

**Revendications**

1. Une composition, stable en l'absence d'humidité, consistant essentiellement en (A) un oligomère de siloxanes ayant au moins une liaison azote-hydrogène, dans lequel les motifs contenant la liaison azote-hydrogène sont choisis dans le groupe consistant en

$$OSi(R)C_mH_{2m}NR'_2, \quad O_{0,5}Si(R_2)C_mH_{2m}NR'_2,$$

$$OSi(R)C_mH_{2m}NR'C_nH_{2n}NR'_2, \quad O_{0,5}Si(R_2)C_mH_{2m}NR'C_nH_{2n}NR'_2,$$

$$O_{1,5}SiC_mH_{2m}NR'_2, \quad O_{1,5}SiC_{m,}H_{2m}NR'C_nH_{2n}NR'_2,$$

$$OSi(R)C_mH_{2m}C(=O)NR'_2, \quad O_{0,5}Si(R_2)C_mH_{2m}C(=O)NR'_2,$$

$$OSi(R)C_mH_{2m}C(=O)NR'C_nH_{2n}C(=O)NR'_2, \quad O_{0,5}Si(R_2)C_mH_{2m}C(=O)NR'C_nH_{2n}C(=O)NR'_2,$$

$$O_{1,5}SiC_mH_{2m}C(=O)NR'_2, \quad O_{1,5}SiC_mH_{2m}C(=O)NR'C_nH_{2n}C(=O)NR'_2,$$

et leurs mélanges, où R est choisi dans le groupe formé par les radicaux hydrocarbonés monovalents, les radicaux hydrocarbonés monovalents substitués et l'hydrogène, R' est choisi dans le groupe formé par l'hydrogène, les radicaux hydrocarbonés monovalents et les radicaux hydrocarbonés monovalents substitués; où au moins un R' par motif siloxy est de l'hydrogène; et m et n ont des valeurs de 1 à 4, les autres motifs de l'oligomère de siloxanes étant choisis dans le groupe formé par $RSiO_{3/2}$, $R_2SiO$ et $R_3SiO_{1/2}$ où R est tel que défini ci-dessus, et (B) un additif anhydre, facilement hydrolysable, choisi dans le groupe constitué des titanates, zirconates, vanadates, germanates et leurs mélanges.

2. Une composition telle que définie dans la revendication 1, dans laquelle l'additif (B) est un titanate.

3. Une composition telle que définie dans la revendication 2, dans laquelle les motifs contenant la liaison azote-hydrogène présents dans l'oligomère de siloxanes (A) sont

$$OSi(R)C_mH_{2m}NR'C_nH_{2n}NR'_2,$$

où R est un radical hydrocarboné monovalent, R′ est de l'hydrogène, *m* a une valeur de 3 ou 4, et *n* a une valeur de 2; et le titanate (B) est un titanate d'alkyle ayant la formule générale $Ti(OD)_4$ où D est un radical alkyle.

4. Une composition telle que définie dans la revendication 3, dans laquelle l'oligomère de siloxanes (A) a la formule générale:

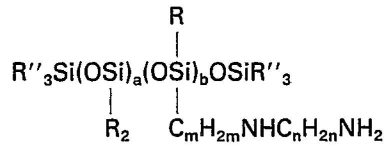

dans laquelle R est un radical alkyle; R″ est choisi dans le groupe constitué des radicaux hydrocarbonés monovalents et des radicaux hydrocarbonés monovalents substitués, et *a*+*b* a une valeur de 1 à 2000, *a* ayant une valeur de 0 à 1999 et *b* ayant une valeur de 1 à 2000.

5. Une composition telle que définie dans la revendication 4, dans laquelle l'oligomère de siloxanes (A) est choisi dans le groupe constitué par

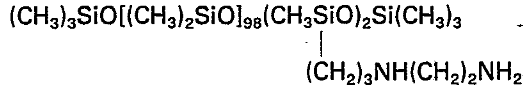

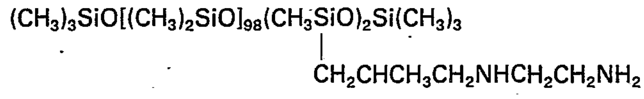 et

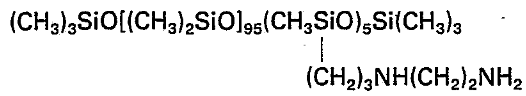 et

le radical alkyle D du composant (B) est choisi dans le groupe constitué des radicaux isopropyle, 2-éthylhexyle et n-butyle.

6. Un procédé pour fixer et conditionner simultanément les cheveux consistant:

(I) à imposer aux cheveux une disposition souhaitée;
(II) à enduire les cheveux d'une composition selon l'une quelconque des revendications 1 à 5; et
(III) à exposer les cheveux enduits à l'air.